# EUROPÄISCHE PATENTSCHRIFT

(11) **EP 3 019 869 B1**
(45) Veröffentlichungstag und Bekanntmachung des Hinweises auf die Patenterteilung: **09.12.2020**
(21) Anmeldenummer: 14750140.7
(22) Anmeldetag: 25.06.2014
(51) Int. Cl.: G01N 33/543, A61K 39/00

(54) **VERFAHREN ZUR QUANTITATIVEN CHARAKTERISIERUNG VON SUBSTANZEN HINSICHTLICH IHRER BINDUNGSEIGENSCHAFTEN AN AMYLOID-BETA (ABETA)-KONFORMERE**
METHOD FOR QUANTITATIVE CHARACTERIZATION OF SUBSTANCES FOR THEIR BINDING TO AMYLOID-BETA (ABETA) CONFORMERS
PROCÉDÉ DE CARACTÉRISATION QUANTITATIVE DE SUBSTANCES QUANT À LEURS PROPRIÉTÉS DE LIAISON À DES CONFORMÈRES DE BETA-AMYLOÏDE

(30) Priorität: 12.07.2013 DE 102013011636
(43) Veröffentlichungstag der Anmeldung: 18.05.2016
(73) Patentinhaber: Forschungszentrum Jülich GmbH, 52425 Jülich (DE)
(72) Erfinder: WILLBOLD, Dieter, 52428 Jülich (DE); GLÜCK, Julian, 40667 Meerbusch (DE); FRENZEL, Daniel, 69121 Heidelberg (DE); NAGEL-STEGER, Luitgard, 40764 Langenfeld (DE); BRENER, Oleksandr, 40591 Düsseldorf (DE)
(74) Vertreter: Fitzner, Uwe
(86) Internationale Anmeldenummer: PCT/DE2014/000320
(87) Internationale Veröffentlichungsnummer: WO 2015/003675

(56) Entgegenhaltungen:
- US-A1- 2012 021 416
- SILKE DORNIEDEN ET AL: "Characterization of a Single-Chain Variable Fragment Recognizing a Linear Epitope of A[beta]: A Biotechnical Tool for Studies on Alzheimer's Disease?", PLOS ONE, Bd. 8, Nr. 3, 1. März 2013 (2013-03-01), Seite e59820, XP55142153, ISSN: 1932-6203, DOI: 10.1371/journal.pone.0059820
- M. STRAVALACI ET AL: "Specific Recognition of Biologically Active Amyloid- Oligomers by a New Surface Plasmon Resonance-based Immunoassay and an in Vivo Assay in Caenorhabditis elegans", JOURNAL OF BIOLOGICAL CHEMISTRY, Bd. 287, Nr. 33, 26. Juni 2012 (2012-06-26), Seiten 27796-27805, XP055142125, ISSN: 0021-9258, DOI: 10.1074/jbc.M111.334979
- OLUBIYI O O ET AL: "Amyloid aggregation inhibitory mechanism of arginine-rich D-peptides", CURRENT MEDICINAL CHEMISTRY, BENTHAM SCIENCE PUBL, NL, Bd. 21, Nr. 12, 1. Januar 2014 (2014-01-01), Seiten 1448-1457, XP009180173, ISSN: 1875-533X
- DANIEL FRENZEL ET AL: "Immobilization of Homogeneous Monomeric, Oligomeric and Fibrillar A[beta] Species for Reliable SPR Measurements", PLOS ONE, Bd. 9, Nr. 3, 3. März 2014 (2014-03-03), Seite e89490, XP055142127, DOI: 10.1371/journal.pone.0089490
- I. Morgado ET AL: "Molecular basis of -amyloid oligomer recognition with a conformational antibody fragment; Supporting information", Proceedings of the National Academy of Sciences, vol. 109, no. 31, 18 July 2012 (2012-07-18), pages 12503-12508, XP55502681, US ISSN: 0027-8424, DOI: 10.1073/pnas.1206433109

### SEQUENCE LISTING

<110> Forschungszentrum Jülich GmbH
<120> Verfahren zur quantitativen charakterisierung von Substanzen hinsichtlich ihrer Bindungseigenschaften an amyloid Beta (ABeta)-Konformere
<130> PT 1.2627
<160> 1
<170> PatentIn version 3.5
<210> 1
   <211> 12
   <212> PRT
   <213> Artificial Sequence
<220>
   <223> D7-Peptid (D-Enantiomer)
<400> 1
   His Thr Arg Phe Glu Tyr Tyr Val Tyr His Met Ser
   1               5                   10

## Beschreibung

Die Erfindung bezieht sich auf ein Verfahren zur quantitativen Charakterisierung von Substanzen hinsichtlich ihrer Bindungseigenschaften an Amyloid-β (Aβ)-Konformere.

### Stand der Technik

Die Biotin-Streptavidin Kopplung von Liganden an einen Biosensor ist Stand der Technik. Ein Ligand wird biotinyliert und an eine Sensoroberfläche, welche mit Streptavidin beladen ist, immobilisert. Zu testende Analyte werden durch die Bindung an den Liganden nachgewiesen. Nachteilig sind für dieses Verfahren Regenerationsschritte erforderlich, die zeitintensiv sind und die nicht-kovalente Bindung des Liganden an der Oberfläche stören.

Dornieden et al. (Dornieden S, Müller-Schiffmann A, Sticht H, Jiang N, Cinar Y, et al. (2013) Characterization of a Single-Chain Variable Fragment Recognizing a Linear Epitope of Aβ: A Biotechnical Tool for Studies on Alzheimers Disease. PLoS ONE 8(3): e59820. doi:10.1371/journal.pone.0059820.) konnten mittels eines ThT Assay zeigen, dass das Antikörperfragment scFv-IC16 die Aβ 1-42 Fibrillenbildung in Aß 1-42 enthaltenden Proben verhindert. In derselben Veröffentlichung wurde beschrieben, ein synthetisches Aβ 1-42 Peptid über sein C-terminales Cystein an die Sensoroberfläche eines CM5-Sensorchip von GE Healthcare zu binden. Oberflächenplasmonresonanz wurde durchgeführt um das Bindungsverhalten des Antikörperfragment scFv-IC16 an Aβ Konformere zu charakterisieren. Das Antikörperfragment scFv-IC16 stellte in diesen Versuchen den Analyten dar.

Stravalaci et al. (J. Biol. Chem. 2012, 287:27796-27805. doi: 10.1074/jbc.MIII.334979 originally published online June 26, 2012) offenbaren einen Antikörper 4G8, welcher als Capture-Agens auf dem Biosensor immobilisiert ist. Dieser bindet spezifisch an A-Beta-Oligomer.

Es existiert kein zugelassenes Medikament für eine ursächliche Behandlung der Alzheimerschen Demenz (AD). Typischerweise findet man in den Gehirnen von AD-Patienten post-mortem Ablagerungen des sogenannten beta-Amyloid-Peptides (Aβ) in Plaques. Es werden verschiedene Formen des Aβ, z.B. Fibrillen, für die Entstehung und das Fortschreiten von AD verantwortlich gemacht. Seit einigen Jahren werden besonders die kleinen Aβ-Aggregate (Aβ-Oligomere) als hauptsächlicher Verursacher für die Entstehung und den Fortschritt der AD verantwortlich gemacht. Eine Reduktion oder vollständige Eliminierung von Aβ-Oligomeren wäre somit das wichtigste Kriterium, um die AD zu heilen oder zu verlangsamen. Aβ-Monomere entstehen ständig in unserem Körper und sind vermutlich per se nicht toxisch. Es wird spekuliert, ob sich Aβ-Monomere abhängig von ihrer Konzentration (die sich letztlich durch Bildungs- und Abbau-Raten im Körper ergibt) zufällig und damit mit zunehmendem Alter immer wahrscheinlicher spontan zu Aβ-Oligomeren zusammen lagern. Einmal entstandene Aβ-Oligomere könnten sich durch einen Prion-ähnlichen Mechanismus dann vermehren und letztlich zur Krankheit führen. Aufgrund dieser Überlegungen sollte es das Ziel einer ursächlichen Behandlung sein, toxische Aβ-Oligomere, möglicherweise aber auch andere Oligomerformen, vollständig zu vernichten und/oder deren Prion-ähnliche Vermehrung zu verhindern.

Ein wichtiger Punkt ist, dass jeder Wirkstoff in einem Tiermodel und in klinischen Studien getestet werden muss. Diese sind sehr zeitintensiv und kostspielig. Von großem Vorteil wäre eine schnelle, zuverlässige und quantitative in vitro-Untersuchung, die sowohl ein Screening verschiedener potentieller Wirkstoffe als auch den Einfluss von Wirkstoffoptimierungen auf das spezifische Bindungsverhalten für unterschiedliche Aβ-Konformere (Monomere, Oligomere, Fibrillen) ermöglicht. Darüber hinaus ist ein direkter Nachweis von Aβ-Oligomeren und/oder anderen Konfomeren verschieden von Monomeren zu jedem gewünschten Zeitpunkt der Messung eine wichtige Bedingung, um als Kontroll-/Qualitätsstandard zu fungieren.

Für quantitative Bindungsanalysen von Substanzen wird derzeit vorrangig die Oberflächenplasmonenresonanz (SPR)-Technologie eingesetzt, da diese neben Bindungsaffinitäten auch Rückschlüsse auf die zugrunde liegenden Assoziations- und Dissoziationsraten zulässt. Für Interaktionsstudien muss einer der beiden Interaktionspartner auf der Sensoroberfläche immobilisiert werden. Dieser wird als Ligand bezeichnet.

Durch die hohe Anfälligkeit von Aβ-Oligomeren zur Strukturänderung ist eine Verwendung als Analyt (injiziertes Molekül) nicht ratsam. Für die Immobilisierung von Molekülen existieren derzeit verschiedene kovalente und nicht-kovalente Strategien. Eine besondere Herausforderung im Fall von Aβ-Oligomeren ist deren hohe Anfälligkeit für strukturelle Änderungen infolge von Veränderungen der umgebenden Lösungsbedingungen, die sich bei kovalenten Kopplungsmethoden nicht vermeiden lassen. Nicht-kovalente Immobilisierungstechniken, sogenannte capture-Verfahren, sind hier die Methode der Wahl. Allerdings besteht hier die Voraussetzung, dass eine erfolgreiche Verknüpfung von geeigneten Aβ-Oligomer-Präparationsmethoden mit nicht-kovalenten Immobilisierungsmethoden gefunden werden muss. Dennoch zeigt sich bei diversen aktuellen Veröffentlichungen ein Trend zur kovalenten Immobilisierung ohne Rücksicht auf dadurch mögliche Strukturänderungen bei immobilisierten Aβ-Oligomeren.

Eine weitere Fehlerquelle bei SPR-Messungen sind Regenerationsschritte zwischen einzelnen Analytinjektionen, da diese ebenfalls strukturelle Änderungen hervorrufen können.

Als gängige Nachweismethode für die erfolgreiche Immobilisierung von Aβ-Oligomeren auf der Sensoroberfläche dient der Nachweis mit Antikörpern. Durch die bivalente Natur der Antikörper ergeben sich nachteilig Aviditätseffekte, die eine vollständige Dissoziation der Moleküle ohne Zugabe von Additiven in einem vertretbaren zeitlichen Umfang nicht erlauben und erneut Regenerationsschritte erfordern.

Durch die beschriebene hohe Anfälligkeit zu Strukturänderungen infolge von wechselnden Lösungsbedingungen, die durch gängige Immobilisierungsprotokolle, Regenerationsschritte zwischen einzelnen Analytinjektionen und Antikörperkontrollinjektionen erforderlich sind, lassen sich Messartefakte bei Interaktionsstudien mit oligomeren Aβ-Formen nicht umgehen. Dies birgt das Risiko, dass erhaltene Messdaten möglicherweise stark fehlerbehaftet sind.

### Aufgabe der Erfindung

Aufgabe der Erfindung ist es ein Verfahren zur quantitativen Charakterisierung von Substanzen hinsichtlich ihrer Bindungseigenschaften an verschiedene Amyloid-β (A β)-Konformere bereit zu stellen. Dies würde ein Screening und die Optimierung zu testender Substanzen z.B. gegen die Alzheimersche Demenz (AD) ermöglichen. Weitere Aufgabe der Erfindung ist es eine Vorrichtung zur Durchführung des Verfahrens bereit zu stellen.

### Lösung der Aufgabe

Die Aufgabe wird gelöst nach dem Verfahren gemäß Patentanspruch 1 . Vorteilhafte Ausgestaltungen hierzu ergeben sich jeweils aus den hierauf rückbezogenen Patentansprüchen.

### Beschreibung der Erfindung

Beansprucht wird ein Verfahren zur quantitativen Charakterisierung von einer Substanz hinsichtlich ihrer Bindungseigenschaften an Amyloid-β (Aβ)-Konformer mit den Schritten:
- Eine Probe, umfassend Aß und N-terminal biotinyliertes Aβ oder ausschließlich N-terminal biotinyliertes Aβ wird vorbereitet und inkubiert, so dass in der Probe verschiedene Konformere des Aβ durch Aggregation vorliegen, wird fraktioniert,
- Biotinyliertes Aβ-Konformer der gewünschten Fraktion, welche nicht Monomere von Aβ umfasst, wird an der Oberfläche eines Substrats mit hoher Affinität zu Biotin immobilisiert,
- Hinzufügen einer Aggregatqualitätskontrollsonde,
- Das Bindungsverhalten einer Aggregatqualitätskontrollsonde an das gewünschte Aβ-Konformer wird durch die Bestimmung der kinetischen und / oder thermodynamischen Parameter aus dem Messsignal abgeleitet,
- wodurch die Qualität der immobilisierten Aggregate überprüft werden kann,
- Hinzufügen einer zu testenden Substanz zum immobilisierten Konformer, wobei das Bindungsverhalten der zu testenden Substanz an das gewünschte Aβ-Konformer durch die Bestimmung der kinetischen und / oder thermodynamischen Parameter aus dem Messsignal abgeleitet wird.

Es wird eine Probe, umfassend monomeres Aβ und monomeres N-terminal biotinyliertes Aβ oder ausschließlich monomeres N-terminal biotinyliertes Aβ vorbereitet und inkubiert, so dass in der Probe verschiedene Konformere des Aβ durch Aggregation gebildet werden.

Die Herstellung der Probe kann für die in vitro Untersuchungen vorzugsweise aus lyophilisierten Ausgangsbestandteilen vorgenommen werden. Die Inkubation der Aβ - Moleküle kann hierzu in HFIP erfolgen, welches im Weiteren wieder abgedampft wird. Sodann wird das Aβ Molekül der Wahl, z. B. Aβ 1-42, in dem gewünschten Mischungsverhältnis an N-Terminal biotinylierten zu nicht-biotinylierten Molekülen in Puffer aufgenommen.

Es können alle eingesetzten Aβ Moleküle zur Herstellung der Probe N-Terminal biotinyliert vorliegen. In diesem Fall kann die Bindung von raumfüllenden Liganden an kontinuierliche Epitope, die den N-terminalen Aminosäurerest enthalten, eingeschränkt sein. Diskontinuierli-che Epitope entstehen aus den Primärstrukturen der Aβ - Moleküle durch Aggregation. Dieser Nachweis der Bindung von Molekülen (Aggregatqualitätskontrollsonde und / oder zu testende Substanz) kann im Fokus des erfindungsgemäßen Verfahrens liegen.

Es können zur Herstellung der Probe aber auch Mischungen aus N-Terminal biotinylierten und nicht-biotinylierten Aβ-Molekülen genutzt werden, und zu Beginn in einem gewünschten Verhältnis zueinander angesetzt werden. Das Verhältnis aus N-Terminal biotinylierten zu nicht-biotinylierten Moleküle sollte vorzugsweise kleiner als 1:1 bis minimal 1:50 betragen. Es ist denkbar, abhängig von der zu immobilisierenden Spezies, auch ein geringeres Verhältnis an N-terminal biotinylierten zu nicht-biotinylierten Molekülen einzusetzen wie zum Beispiel 1:100.

Die N-Terminal biotinylierten Moleküle in einem Aggregat gewährleisten die Immobilisierung auch der nicht-biotinylierten Aβ Moleküle im selben Aggregat an eine Substrat-Oberfläche.

In diesem Fall ist durch das nach gelagerte, quantitative Verfahren auch die Bindung von Aggregatqualitätskontrollsonde und / oder zu testende Substanz an den frei liegenden N-terminalen Primärstrukturen von Aβ Molekülen möglich. Dieser Nachweis der Bindung von Molekülen kann ebenfalls im Fokus des Verfahrens liegen.

Besonders vorteilhaft wird ein Verhältnis von N-Terminal biotinylierten zu nicht-biotinylierten Aβ Molekülen von kleiner gleich 1:10 bis 1:40 verwendet.

In *in vitro* Studien kann hierzu aus synthetisch bereit gestellten N-Terminal biotinylierten und nicht-biotinylierten Aβ Molekülen eines Zulieferers nach jeweiliger Aufnahme in HFIP das gewünschte Verhältnis angemischt werden. Dadurch sind vorteilhaft alle während des Versuchs benutzten Molekülgemische vor der einsetzenden Aggregation bereits im gewünschten Verhältnis biotinyliert.

Um die Immobilisierung von oligomeren Aβ-Formen zu ermöglichen, bei der keine Risiken einer strukturellen Änderungen eingeführt werden, werden demnach N-terminal biotinylierte Aβ Peptide in einem Verhältnis von kleiner gleich 1:10 mit Aβ co-inkubiert.

Vorteilhaft wird durch diesen Schritt eine gezielte Capture-Immobilisierung, insbesondere von oligomeren und höheren Aβ-Formen mit Hilfe von Streptavidin oder Avidin beladenen Sensoroberflächen ermöglicht, ohne zusätzlich erforderliche Änderungen der Lösungsbedingungen.

Die co-inkubierten Peptide aus N-Terminal biotinylierten und nicht-biotinylierten Molekülen einer Fraktion werden hierzu an der Streptavidin (Avidin) beladenen Oberfläche des Substrats immobilisiert. Als Immobilisierungsmechanismus wird somit die hohe Streptavidin (bzw. Avidin) -Biotin-Affinität der beiden Bindungspartner genutzt.

Die Probe umfasst nach der Inkubation die verschiedenen, möglichen Konformere des Aβ, wie z. B. Monomere, Oligomere, Fibrillen, und höhere Konformationsstrukturen des Aβ.

Das Verfahren ist besonders geeignet quantitative Analysen an den stark aggregationsfreudigen Aβ 1-42 Konformeren vorzunehmen, insbesondere an den Aβ 1-42 Oligomeren. Dafür wurde das beanspruchte Verfahren insbesondere entwickelt. Es sind aber selbstverständlich auch andere Aβ Spezies wie z. B. das Aβ 1-40 als immobilisierter Ligand des Verfahrens möglich. Auch der Einsatz von Mischungen aus mehreren unterschiedlichen Aβ Spezies, wie z. B. Aβ 1-40, Aβ 1-42 und pyroglutamyl- Aβ 3-40, ist möglich.

Die Probe mit den verschiedenen Aβ-Konformere wird sodann fraktioniert. In jeder Fraktion werden entsprechend des Fraktionierungsschritts verschiedene Konformere angereichert und können exakt bestimmt werden.

Der Begriff "exakt bestimmt" umfasst einen Kalibrierungsschritt bei der Fraktionierung durch Moleküle bekannter Art und Verhaltens. Nach der Fraktionierung liegt in jeder Fraktion nur eine bestimmte Art an Konformere des Aβ vor, z. B. Oligomere oder Fibrillen und so weiter.

Bei der Dichtegradientenzentrifugation als Fraktionierungsschritt werden die Konformere nach ihrem s-Wert oder Sedimentationskoeffizienten aufgetrennt. Moleküle unterschiedlicher Größe können einen identischen hydrodynamischen Radius besitzen, haben aber dennoch unterschiedliche s-Werte und werden danach auch aufgetrennt. Durch eine Kalibration mit Molekülen von bekanntem s-Wert sind die mittels Dichtegradientenzentrifugation erhaltenen Aβ Konformere exakt nach ihrem s-Wert bestimmt.

Bei der Größenausschlusschromatographie hingegen werden die Fraktionen auf Grund der Größe der Aβ Moleküle gebildet. Diese können im Ergebnis den bekannten Monomeren und Oligomeren zugeordnet werden.

Somit ist jede im Weiteren zu untersuchende Fraktion hinsichtlich des diese beinhaltenden Aβ Konformers exakt bestimmt.

Auf diese Weise wird besonders vorteilhaft bewirkt, dass eine quantitativen Analyse genau eines bestimmten Aβ Konformers ermöglicht wird. Nach Kenntnis der Anmelderin ist dies erstmalig in der vorliegenden Patentanmeldung der Fall.

Als Fraktionierung kommen insbesondere die Dichtegradientenzentrifugation oder auch die Größenausschlusschromatographie in Betracht. Die Dichtegradientenzentrifugation weist den ganz besonderen Vorteil auf, dass hiermit alle Aβ Konformere in einem einzigen Verfahrensschritt voneinander getrennt werden können. Größenausschlusschromatographie kann vorgenommen werden, sofern für die quantitative Analyse die Aβ Oligomere oder Monomere herangezogen werden sollen. Größenausschlusschromatographie kann hingegen keine in der mobilen Phase unlöslichen Aβ-Fibrillen von anderen Aβ Konformeren trennen.

Durch den Einsatz eines Dichtegradientenzentrifugationsschritts vor der Immobilisierung, bei dem auf einen vorgeformten Dichtegradienten oligomere oder höhere Aβ-Formen aufgeschichtet und die darin enthaltenen Aggregat-Partikel entsprechend ihres s-Werts durch Ultrazentrifugation getrennt werden, lassen sich im Laufe dieser Zentrifugation unterschiedliche Aβ-Aggregate (Oligomere und Fibrillen oder amorphe Aggregate) nach ihrem unter anderem von der Partikelgröße abhängigen Sedimentationskoeffizienten voneinander trennen und fraktionieren. Die Fraktion mit dem gewünschten Aβ Konformer kann anschließend direkt auf eine Streptavidin beladene Sensoroberfläche zur Immobilisierung injiziert werden.

Das Aβ Konformer einer bestimmten Fraktion, welche nicht das Monomer umfasst, wird dadurch an der Oberfläche eines Substrats mit hoher Affinität zu Biotin immobilisiert. Als Substrate können generell alle Streptavidin oder Avidin beschichteten Substrate, und insbesondere die Biosensoren für die Oberflächenplasmonresonanz oder die der Biolayer-Interferometrie genutzt werden.

Auf der sensitiven Oberfläche der Biosensoren für die Biolayer-Interferometrie und / oder der Biosensoren für die Oberflächenplasmonresonanz werden auf diese Weise genau definierte Aβ Konformere immobilisiert. Hierdurch wird vorteilhaft bewirkt, dass erstmalig die Biosensoren für die Biolayer-Interferometrie oder die Biosensoren für die Oberflächenplasmonresonanz mit genau einem einzigen, exakt bestimmten Konformer und nicht wie bisher im Stand der Technik üblich, mit einer Mischung verschiedener Aβ Konformere beschichtet sind.

Die quantitave Analyse sieht sodann vor, eine Aggregatqualitätskontrollsonde, die den N-Terminalen Bereich des Konformers spezifisch erkennt zum immobilisierten Aβ Konformer hinzugegeben. Dabei ist besonders vorteilhaft die Bindung der Aggregatqualitätskontrollsonde an die N-Terminalen biotinylierten Bereiche der Moleküle unterbunden, denn auf Grund der sterischen Begebenheiten des Biotin kann die zur Detektion vorgesehene Aggregatqualitätskontrollsonde die Bindungsregion am N-Terminalen Bereich der Moleküle nicht erkennen.

Es wird somit besonders vorteilhaft die Kinetik der Bindung der Aggregatqualitätskontrollsonde an das immobilisierte Aβ Konformer bestimmt. Je nach Ansatz mit oder ohne nichtbiotinylierte Moleküle werden auch diskontinuierliche Epitope erfasst.

Dieser Ansatz macht die Immobilisierungseffizienz des Nicht Monomeren Konformers aus einer Fraktion mit der Bindung der Aggregatqualitätskontrollsonde quantitativ bestimmbar.

Das Verfahren zeichnet sich unter anderem dadurch aus, dass eine Immobilisierung von co-inkubierten Peptiden mit einem Antikörperfragment wie z. B. dem scFv-IC16 als Aggregatqualitätskontrollsonde kontrolliert werden kann. Es ist denkbar, einen anderen spezifisch die N-Terminalen Bereiche erkennenden Antikörper zu verwenden.

Der Nachweis einer erfolgreichen Immobilisierung von oligomeren oder multimeren Aβ Konformeren erfolgt dabei mit Hilfe eines Antikörperfragments, einem sogenannten single chain variable fragment (scFv-IC16), welches spezifisch den N-terminalen Bereich von Aβ erkennt. Durch die Biotinylierung von Aβ am N-terminalen Bereich wird die Bindung des scFv-IC16 inhibiert. Besonders vorteilhaft wird mit dem erfindungsgemäßen Verfahren und der Vorrichtung zur Durchführung des Verfahrens dennoch ein positives Bindungssignal mit scFv-IC16 oder anderen Antikörpern nach der Immobilisierung von oligomeren und anderen multimeren Aβ-Formen erhalten.

Dies ist somit ein direkter Nachweis für nicht monomere Aβ Konformere, da bei monomerem N-terminal biotinyliertem Aβ kein Signal auftreten kann. Darüber hinaus besitzt scFv-IC16 vorteilhaft ein Dissoziationsverhalten, das nach einer kurzen Wartezeit eine vollständige Dissoziation ermöglicht. Hierzu muss besonders vorteilhaft das Antikörperfragment scFv-IC16 lediglich mit Puffer eluiert werden.

Mit dem Verfahren sind vorteilhaft die maximal möglichen Bindungsstellen quantitativ bestimmbar. Da sich der Komplex aus gebundenem Antikörperfragment scFv-IC16 an einem nicht-biotinylierten Aβ durch eine hohe Dissoziationskonstante K_{D} auszeichnet, ist vorteilhaft gewährleistet, dass danach andere Bindungspartner des Aβ quantitativ untersucht werden können, z. B. zu testende Substanzen wie D3 oder D3D3 oder D7.

Das Verfahren ist hierauf nicht beschränkt. Es werden vielmehr alle Bindungsmöglichkeiten der immobilisierten, nicht-biotinylierten Peptide durch das Antikörperfragment scFv-IC16 nachgewiesen und als Kontrolle für die quantitative Analyse der zu testenden Substanz angesetzt. Es sind nicht nur die nach der Primärstruktur insgesamt möglichen Bindungen auf der Sensor-Oberfläche nachweisbar, sondern auch weitere sekundäre Bindungsmöglichkeiten, welche durch die multimere Konformationen der angeordneten Peptide hervorgerufen werden.

Eine zum immobilisierten Konformer hinzugefügte zu testende Substanz kann hinsichtlich der Kinetik der Bindung an das immobilisierte Konformer bestimmt werden. Hierdurch sind Wirkstoffanalysen möglich.

Dabei wird vorteilhaft auch die Bindungskinetik von der zu testenden Substanz an Aβ mit der Bindungskinetik von der Aggregatqualitätskontrollsonde verglichen, wobei die Aggregatqualitätskontrollsonde und insbesondere ein Antikörperfragment scFv-IC16 als Positivkontrolle dient.

Die Ergebnisse können durch Oberflächenplasmonresonanz oder durch Biolayer-Interferometrie erhalten werden. Bei der Oberflächenplasmonresonanz werden Regenerationsschritte zwischen einzelnen Analytinjektionen umgangen, indem die Interaktionsstudien mit Hilfe der "single-cycle kinetics" Messmethode durchgeführt werden. Hierdurch werden vorteilhaft verschiedene Analytkonzentrationen direkt nacheinander injiziert

Eine ebenfalls offenbarte Vorrichtung zur Durchführung eines derartigen Verfahrens ist dadurch gekennzeichnet, dass an einer Substrat-Oberfläche mit hoher Affinität zu Biotin ein bestimmtes Aβ-Konformer, welches nicht Monomere Aβ umfasst, immobilisiert ist.

Die Vorrichtung kann ein käuflich erhältlicher Streptavidin oder Superstreptavidin oder Avidin aufweisender Biosensor für die Biolayer-Interferometrie oder ein entsprechender Biosensor für die Oberflächenplasmonresonanz sein.

Eine zu testende Substanz, die zum auf diese Weise immobilisierten Peptid auf die Substrat-Oberfläche hinzu gegeben wird, ist dann durch eine Oberflächenplasmonresonanz oder durch eine Biolayer-Interferometrie hinsichtlich der Kinetik und Thermodynamik des Bindungsverhaltens an das Peptid exakt quantitativ zu untersuchen.

Zusammengefasst besteht die Erfindung in der gezielten Verknüpfung von verschiedenen Techniken: der Präparation der zu immobilis' ierenden Aβ Konformere, vorzugsweise mittels Größenausschlusschromatographie oder Dichtegradientenzentrifugation, der anschließenden Immobilisierung mittels Biotin-Streptavidin (-Avidin) auf einer Substrat-Oberfläche, dem spezifischen Nachweis von oligomeren oder multimeren Aβ Konformeren mit Hilfe spezifi- scher Antikörper, wie beispielweise scFv-1C16 und der Verwendung der single-cycle kinetics Messmethode im Falle der Oberflächenplasmonresonanz.

Der apparative Aufwand insgesamt ist in Hinblick auf das erzielte Ergebnis gering, denn man benötigt nur eine Ultrazentrifuge und ein Instrument mit dem sich die Kinetik der Protein-Wechselwirkungen studieren lassen, bei dem eine Immobilisierung von Molekülen erforderlich bzw. möglich ist.

### Ausführungsbeispiele

Im Weiteren wird die Erfindung an Hand von Ausführungsbeispielen und der beigefügten Figuren näher erläutert, ohne dass es hierdurch zu einer Beschränkung der Erfindung kommen soll.

Es zeigen:

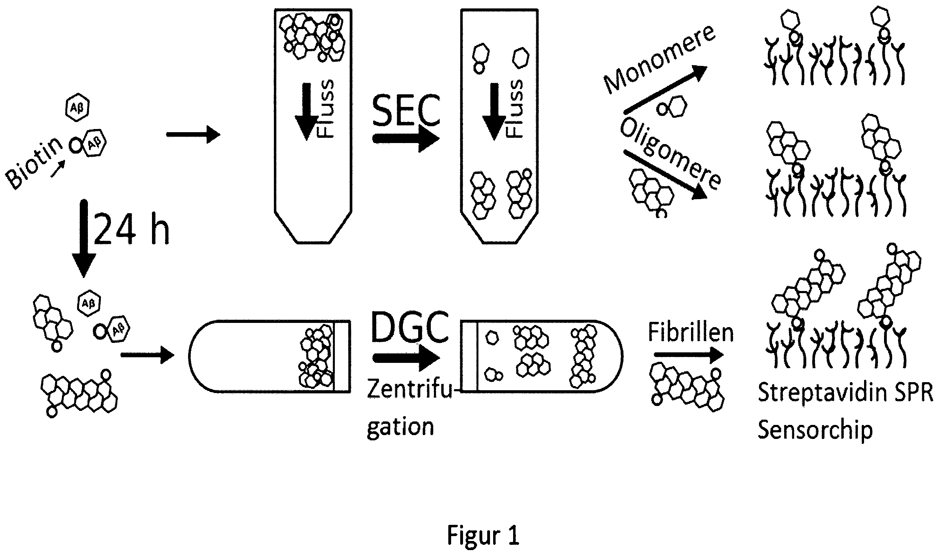

- Figur 1:: Fließschema der Probenpräparation monomerer, oligomerer und fibrillärer Aβ(1-42) Formen mit anschließender Immobilisierung auf SPR-Sensoroberflächen.
- Figur 2:: Ergebnisse zur Immobilisierung von monomeren, oligomeren und fibrillären Aβ(1-42) Formen auf Streptavidin-beladenen SPR-Sensoroberflächen.
- Figur 3:: Darstellung von Sensorgrammen, die mit dem scFv-IC16 und verschiedenen Aβ(1-42) Konformeren erhalten wurden.
- Figur 4:: Darstellung von Sensorgrammen die mit dem Peptid D7 und verschiedenen Aβ(1-42) Konformeren erhalten wurden,das heißt nach Bindung an Aβ(1-42) Monomere (A), Oligomere (B) und Fibrillen (C)

Um eine homogene Probenpräparation zu gewährleisten, wurden Monomere und Oligomere mit Hilfe einer Größenausschlusschromatographie (SEC), und Fibrillen mit Hilfe einer Dichtegradientenzentrifugation von anderen Formen getrennt. Die Immobilisierung auf Streptavidin-beladenen SPR-Sensoroberflächen erfolgte durch die Verwendung von biotinyliertem Aβ(1-42), wobei Oligomere und Fibrillen aus einem 1:10 Verhältnis von biotinyliertem Aβ(1-42) mit nicht-biotinyliertem Aβ(1-42) zusammengesetzt werden.

Die lyophilisierten Proben von N-terminal biotinyliertem Aβ(1-42) und nicht-biotinyliertem Aβ(1-42) wurden jeweils getrennt voneinander in 100 % Hexafluoroisopropanol (HIFP) gelöst und über Nacht bei Raumtemperatur inkubiert. Anschließend wurden die für ein 1:10 Verhältnis benötigten Volumina vereint und das HFIP mit Hilfe eines Konzentrators abgedampft.

Für die Präparation von monomerem und oligomerem Aβ(1-42) wurde das zuvor erhaltene Pellet in dem Puffer der Größenausschlusschromatographie (50 mM Natriumphosphatpuffer, 150 mM NaCl, 0,6 % Tween 20, pH 7,4) aufgenommen, kurz für 30 Sekunden bei 15500 g zentrifugiert, um unlösliches Material zu sedimentieren.

Für die Präparation von fibrillärem Aβ(1-42) wurden die nach dem Abdampfen erhaltenen Pellets in 10 mM Natriumphosphatpuffer pH 7,4 gelöst und für 24 Stunden bei 25 °C bei 600 rpm inkubiert.

Der Überstand nach dem Zentrifugationsschritt zum Ende der Präparation von monomerem und oligomerem Aβ(1-42) wurde anschließend direkt auf eine Superdex 75 10/300 GL (GE Healthcare) Säule zur chromatographischen Trennung aufgetragen. Die Flussrate während der Elution mit Größenausschlusschromatographiepuffer betrug 0,8 ml/min. Monomere eluierten bei ∼14 ml, wohingegen Oligomere bei ∼8 ml eluierten.

Die Vorbereitung des Dichtegradienten erfolgte durch nachfolgendes Überschichten der Dichtegradientenlösung (Iodixanol verdünnt in 10 mM Natriumphosphatpuffer pH 7,4) in Konzentrationen von 50 % (260 µl), 40 % (260 µl), 30 % (260 µl), 20 % (260 µl), 10 % (260 µl), und 5 % (100 µl) (v/v). Im Anschluss wurden die Proben auf den Dichtegradienten pipettiert und die Zentrifugation für 3 Stunden bei 4 °C und 259000 g durchgeführt. Nun wurden von dem Dichtegradienten insgesamt 14 Fraktionen zu je 140 µl von oben nach unten entnommen. Monomere sind in den ersten Fraktionen zu finden, Oligomere in den Fraktionen 5 und 6 und Fibrillen befinden sich typischerweise in Fraktionen 11 - 13.

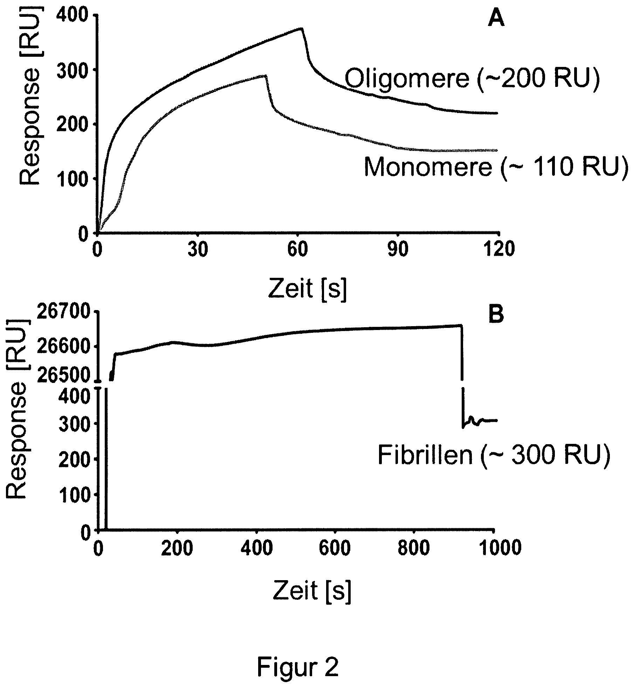

In Figur 2 sind die response units (RU) während der Immobilisierungsinjektion auf der γ-Achse gegen die Zeit in Sekunden auf der X-Achse aufgetragen. Die Figur 2 zeigt dabei in den jeweiligen Sensorgrammen die erhaltenen response units (RU) während der Immobilisierungsinjektion. Das heißt, dass die Aβ 1-42 Moleküle auf dem Streptavidin-beladenen Chip inkubiert werden. In den hier dargestellten Immobilisierungsreaktionen wurden -110 RU Monomere, ∼200 RU Oligomere und ∼300 RU Fibrillen immobilisiert und nach Ende der Immobilisierungsreaktion stellt sich eine stabile Basislinie ein. Dies zeigt, dass die verschiedenen Formen mittels der Biotin - Streptavidin Interaktion erfolgreich immobilisiert werden können und durch die Einstellung einer stabilen Basislinie sind nun die Oberflächen für Interaktionstudien verwendbar.

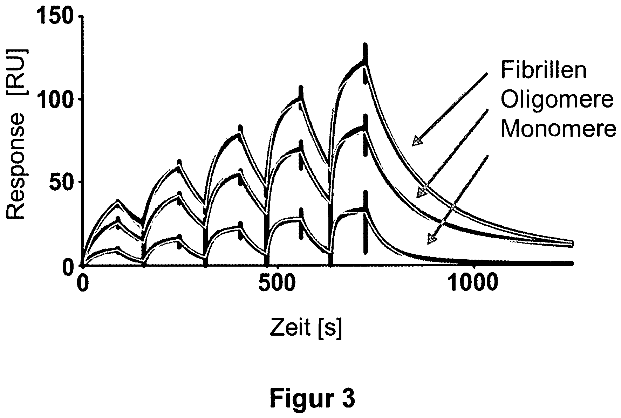

Figur 3 zeigt Sensorgramme, die durch die Injektion des single-chain variable fragment (scFv) IC16 in den Konzentrationen 5, 2.5, 1.25, 0.625, 0.3125 µM über Sensoroberflächen mit immobilisierten Aß(1-42) Monomeren, Oligomeren und Fibrillen erhalten wurden. Die Monomere sind anders als die Oligomere und Fibrillen (N-Terminal) C-Terminal biotinyliert. Es wurden die Response Units (Y-Achse) gegen die Zeit in Sekunden (X-Achse) aufgetragen, wobei die schwarzen Kurven die doppelt-referenzierten experimentellen Daten und die grauen überlagerten Kurven die Fits repräsentieren. Damit zur Charakterisierung des scFv-IC16 Messungen mit biotinyliertem Aβ(1-42) möglich sind, wurde hier C-terminal biotinyliertes Aβ(1-42) verwendet. Für den Fit an Monomere wurde ein 1:1 Langmuir Bindungsmodell genutzt und Oligomere und Fibrillen wurden mit einem heterogenen Bindungsmodell gefittet, das zwei verschiedenen Bindungsstellen auf der Sensoroberfläche berücksichtigt. Es zeigt sich eindeutig, dass die Fits eine sehr gute Übereinstimmung mit den experimentellen Daten aufweisen. Dies ist ein direkter Nachweis für die erfolgreiche Immobilisierung der verschiedenen Formen und die jeweils homogene Oberfläche. Gleichzeitig deutet dies an, dass die ermittelten kinetischen Parameter für die jeweiligen Interaktionen des scFv-IC16 an verschiedene Formen auf einer robusten Charakterisierung beruht.

Die nachfolgende tabellarische Übersicht zeigt die erhaltenen kinetischen Parameter für Aβ(1-42) Monomere, Oligomere und Fibrillen aus Figur 3. *k*ₐ repräsentiert die Assoziationsrate, *k*_{d} die Dissoziationsrate, *K*_{D} die Dissoziationskonstante und Rₘₐₓ das maximal erreichbare Messsignal des Antikörperfragments scFv-IC16 an die jeweilige Bindungsstelle des Konfomers in Response Units.

| | Monomere | Monomere^{‡} | Oligomere^{‡} | Fibrillen^{‡} |
|---|---|---|---|---|
| *k*ₐ₁^{[a]} | 2.27*10⁴ | 2.16*10⁴ | 2.66*10⁴ | 2.96*10⁴ |
| *k*_{d1}^{[b]} | 1.74*10⁻² | 2.03*10⁻² | 0.98*10⁻² | 0.92*10⁻² |
| *K*_{D1}^{[cl} | 7.69*10⁻⁷ | 9.36*10⁻⁷ | 3.70*10⁻⁷ | 3.12*10⁻⁷ |
| *kₐ₂*^{[a]} | - | 4.80*10⁴ | 1.03*10² | 4.54*10² |
| *k*_{d2}^{[b]} | - | 4.78*10⁻⁹ | 5.76*10⁻⁴ | 1.93*10⁻³ |
| *K*_{D2}^{[c]} | - | 9.90*10⁻¹⁴ | 5.60*10⁻⁶ | 4.26*10⁻⁶ |
| χ² | 4.1 | 2.4 | 3.0 | 1.2 |
| Rₘₐₓ₁^{[d]} | 36.6 | 36.2 | 69.1 | 89.3 |
| Rₘₐₓ₂^{[d]} | - | 1.6 | 206.1 | 143.1 |

| | | | | |
|---|---|---|---|---|
| ^{‡}gefittet mit einem heterogenen Bindungsmodell [a] M⁻¹s⁻¹ [b] s⁻¹ [c] M [d] RU | | | | |

Der Index a1 und d1 bezieht sich auf die Assoziations- bzw. Dissoziationsrate des Antikörperfragments scFv-IC16 an das jeweilige freiliegende Epitop des Konformers. Der Index a2 und d2 bezieht sich hingegen auf die Assoziations- bzw. Dissoziationsrate des Antikörperfragments scFv-IC16 an diskontinuierliche Epitope des jeweiligen Konformers. Analog beziehen sich die Indizes D1 und D2 auf die Dissoziationskonstanten an das kontinuierliche und das diskontinuierliche Epitop.

### Zu testende Substanz:

| | |
|---|---|
| D7 | htrfeyyvyhms, gemäß SEQ ID NO: 1 |

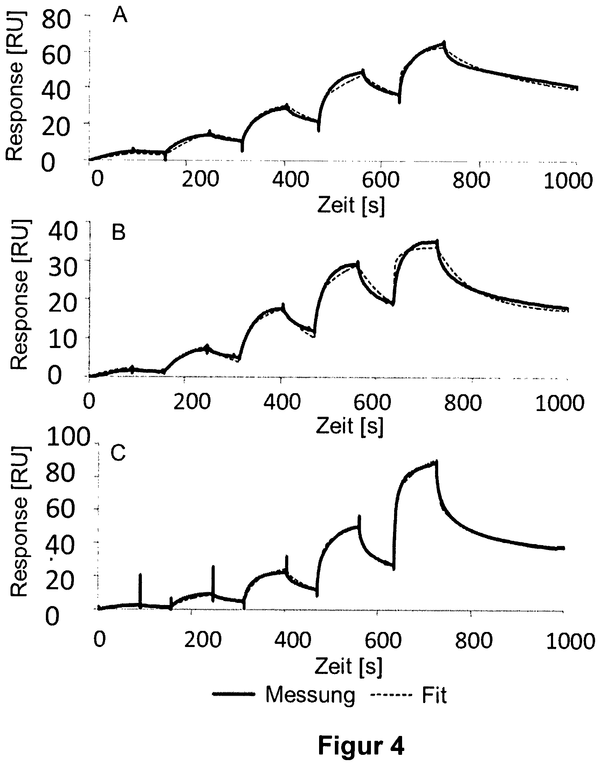

Das D-Enantiomere Peptid D7 wurde durch eine Spiegelbild-Phagendisplayselektion identifiziert und mit dem oben beschriebenen Verfahren hinsichtlich der Bindungseigenschaften exemplarisch als zu testende Substanz untersucht. Abweichend von den Messdaten der Figur 3 wurden in diesem Fall alle verschiedenen Aβ(1-42) Konformere (Monomere, Oligomere und Fibrillen) mittels Dichtegradientenzentrifugation präpariert und auf der Sensoroberfläche in verschiedenen Mengen immobilisiert. Bei den Monomeren wurden 1423 RU immobilisiert, bei den Oligomeren 1356 RU und bei den Fibrillen 1324 RU. Die erhaltenen Bindungsdaten der kinetischen Messungen mit Peptid D7 sind in Figur 4 dargestellt. Wie zuvor in Figur 3 wurde die Messung mit der single-cycle kinetics Methode durchgeführt und die erhaltene Response in Response Units auf der Y-Achse gegen die Zeit in Sekunden auf der X-Achse dargestellt. Die durchgehende schwarze Linie entspricht den experimentellen Daten der Injektionen von 12.5, 3.13, 0.781, 0.195, 0.0488 µM D7, wobei mit der geringsten Konzentration begonnen und mit der höchsten Konzentration aufgehört wurde. Es zeigt sich hier sehr deutlich, dass das oben beschriebene Verfahren dazu geeignet ist eine zu testende Substanz D7 hinsichtlich der Bindungseigenschaften an verschiedene Aβ(1-42) Konformere zu untersuchen. Darüber hinaus dient dieses Anwendungsbeispiel als Nachweis, dass sich ebenfalls Monomere und Oligomere nach der Dichtegradientenzentrifugation identisch zu den Fibrillen immobilisieren lassen und nachfolgend für Bindungsstudien zur Verfügung stehen.

Die nachfolgende tabellarische Übersicht zeigt die erhaltenen kinetischen Parameter für Aβ(1-42) Monomere, Oligomere und Fibrillen aus Figur 4. *k*ₐ repräsentiert die Assoziationsrate, *k*_{d} die Dissoziationsrate, *K*_{D} die Dissoziationskonstante und Rₘₐₓ das maximal erreichbare Messsignal des Peptids D7 an die jeweilige Bindungsstelle des Konfomers in Response Units. Die Sensorgramme für Monomere und Oligomere Aβ(1-42) Konformere wurde mit einem heterogenen Bindungsmodell gefittet, das zwei verschiedene Bindungsstellen berücksichtigt. Im Fall der Aβ(1-42) Fibrillen wurde ein Bindungsmodell mit drei verschiedenen Bindungsstellen berücksichtig. Die Indizes a1, a2, a3, d1, d2, d3, D1, D2, D3, max1, max2, max3 geben an zu welcher der gefundenen Teilreaktionen die Parameter gehören.

**Tabelle 2: Kinetik der Bindung zwischen D7 und Aβ 1-42 Monomere, Oligomere und Fibrillen**

| | Monomere^{‡} | Oligomere^{‡} | Fibrillen^{‡} |
|---|---|---|---|
| *k*ₐ₁^{[a]} | 2416 | 4521 | 1,82*10⁴ |
| *k*_{d1}^{[b]} | 3,64*10⁻⁴ | 4,02*10⁻⁴ | 0,2618 |
| *K*_{D1}^{[c]} | 1,51*10⁻⁷ | 8,88*10⁻⁸ | 1,44*10⁻⁵ |
| *k*ₐ₂^{[a]} | 1,00*10⁵ | 6,62*10⁴ | 1318 |
| *k*_{d2}^{[b]} | 0,009276 | 0,01527 | 1,93*10⁻⁴ |
| *K*_{D*2*}^{[c]} | 9,26*10⁻⁸ | 2,31*10⁻⁷ | 1,47*10⁻⁷ |
| *k*ₐ₃^{[a]} | - | - | 3,23*10⁴ |
| *k*_{d3}^{[b]} | - | - | 0,0125 |
| *K*_{D3}^{[c]} | - | - | 3,88*10⁻⁷ |
| χ² | 1,32 | 0,5 | 0,62 |
| Rₘₐₓ₁^{[d]} | 37,35 | 19,24 | 57,27 |
| Rₘₐₓ₂ ^{[d]} | 27,5 | 14,74 | 45,94 |
| Rₘₐₓ₃^{[d]} | - | - | 25,79 |

Das Verfahren wird erneut angewendet. Nach der Abdissoziation des D7 von dem Aβ - Konformer wird erneut mit der Aggregatqualitätskontrollsonde die Oberfläche des Biosensors hinsichtlich der kinetischen und / oder thermodynamischen Parameter aus dem Messsignal überprüft wird und Aussagen über die Qualität der Oberfläche des Sensors getroffen. Mit dem erfindungsgemäßen Verfahren ist also gewährleistet, dass nur Oberflächen von Biosensoren ausreichend guter Qualität für weitere zu testende Substanzen verwendet werden.

Hierdurch wird vorteilhaft der Verbrauch gesenkt gleichbleibend hohe Qualität des Sensors für nachgelagerte Studien gewährleistet.

## Patentansprüche

1. Verfahren zur quantitativen Charakterisierung einer Substanz hinsichtlich ihrer Bindungseigenschaften an Amyloid-β (Aβ)-Konformere mit den Schritten:
- Eine Probe, umfassend Aβ und N-terminal biotinyliertes Aβ oder ausschließlich N-terminal biotinyliertes Aβ wird vorbereitet und inkubiert, so dass in der Probe verschiedene Konformere des Aβ durch Aggregation vorliegen, wird fraktioniert,
- Biotinyliertes Aβ-Konformer der gewünschten Fraktion, welche nicht Monomere von Aβ umfasst, wird an der Oberfläche eines Substrats mit hoher Affinität zu Biotin immobilisiert,
- Hinzufügen einer Aggregatqualitätskontrollsonde,
- Das Bindungsverhalten einer Aggregatqualitätskontrollsonde an das gewünschte Aβ-Konformer wird durch die Bestimmung der kinetischen und / oder thermodynamischen Parameter aus dem Messsignal abgeleitet,
- wodurch die Qualität der immobilisierten Aggregate überprüft werden kann,
- Eine zu testende Substanz zum immobilisierten Konformer hinzugegeben wird und das Bindungsverhalten der zu testenden Substanz an das gewünschte Aβ-Konformer durch die Bestimmung der kinetischen und / oder thermodynamischen Parameter aus dem Messsignal abgeleitet wird.

2. Verfahren nach Anspruch 1, **dadurch gekennzeichnet, dass** eine Probe mit Aβ-Konformeren aus Zellkultur oder durch Entnahme aus einem Körper erhalten wurde.

3. Verfahren nach einem der vorhergehenden Ansprüche, **gekennzeichnet durch** eine Dichtegradientenzentrifugation als Fraktionierungsschritt.

4. Verfahren nach vorherigem Anspruch, **gekennzeichnet durch** eine Kalibrierung der Dichtegradientenzentrifugation.

5. Verfahren nach einem der vorhergehenden Ansprüche, **dadurch gekennzeichnet, dass** ein Verhältnis von N-Terminal biotinylierten zu nicht-biotinylierten Molekülen von 1 bis 1:100 gewählt wird.

6. Verfahren nach einem der vorhergehenden Ansprüche, **gekennzeichnet durch** Wahl des Antikörperfragments scFv-1C16 als Aggregatqualitätskontrollsonde.

7. Verfahren nach einem der vorhergehenden Ansprüche, **gekennzeichnet durch** Wahl der Versuchsparameter bei der die Aggregatqualitätskontrollsonde von Aβ abdissoziiert.

8. Verfahren nach vorherigem Anspruch, **gekennzeichnet dadurch, dass** die zu testende Substanz von dem Aß Konformer abdissoziiert.

9. Verfahren nach vorherigem Anspruch, **gekennzeichnet dadurch, dass** die Aggregatqualitätskontrollsonde erneut zum Konformer hinzugefügt wird und die Oberfläche des Substrats hinsichtlich der Qualität für weitere Bindungsstudien überprüft wird.

10. Verfahren nach einem der vorhergehenden Ansprüche, **gekennzeichnet durch** Durchführung einer Oberflächenplasmonresonanz oder einer Biolayer-Interferometrie zur Bestimmung der kinetischen und / oder thermodynamischen Parameter.

11. Verfahren nach einem der vorhergehenden Ansprüche, wobei der erste Schritt entfallen kann, sofern man die gewünschten Aβ-Konformere auf andere Weise, z. B. aus einem anderen Experiment erhält.

## Claims

1. Method for quantitative characterization of a substance for its binding properties to amyloid-β [Aβ] conformers with the steps:
- fractionation of a sample, comprising Aβ and N-terminal biotinylated Aβ or solely N-terminal biotinylated Aβ is prepared and incubated so that different conformers of the Aβ are present in the sample by aggregation,
- biotinylated Aβ conformer of the desired fraction, which does not comprise monomers of Aβ, is immobilized on the surface of a substrate having a high biotin affinity,
- addition of an aggregate quality control probe,
- the binding characteristics of an aggregate quality control probe to the desired Aβ conformer are derived from the measurement signal by determination of the kinetic and/or thermodynamic parameters,
- whereby the quality of the immobilized aggregates can be verified,
- a substance to be tested is added to the immobilized conformer and the binding characteristics of the substance to be tested to the desired Aβ conformer are derived from the measurement signal by determination of the kinetic and/or thermodynamic parameters,

2. Method according to claim 1, **characterized in that** a sample with Aβ conformers has been obtained from cell culture or by removal from a body.

3. Method according to one of the preceding claims, **characterized by** a density gradient centrifugation as fractionation step.

4. Method according to the preceding claim, **characterized by** a calibration of the density gradient centrifugation.

5. Method according to one of the preceding claims, **characterized in that** a ratio of N-terminal biotinylated to non biotinylated molecules is chosen from 1 to 1:100.

6. Method according to one of the preceding claims, **characterized by** the choice of the antibody fragment scFv-1C16 as aggregate quality control probe.

7. Method according to one of the preceding claims, **characterized by** choice of test parameters which the aggregate quality control probe dissociates from Aβ.

8. Method according to the preceding claim, **characterized in that** the sub stance to be tested dissociates from the Aβ conformer.

9. Method according to the preceding claim, **characterized in that** the aggregate quality control probe again is added to the conformer and the surface of the substrate is tested with regard to the quality for further binding studies.

10. Method according to one of the preceding claims, **characterized by** performing a surface plasmon resonance or a biolayer interferometry for the determination of the kinetic and/or thermodynamic parameters.

11. Method according to one of the preceding claims, whereby the first step can be omitted, provided that the desired Aβ conformers can be obtained in another way, for example from another experiment.

## Revendications

1. Procédé de caractérisation quantitative d'une substance quant à ses pro priétés de liaison aux [Aβ]-conformères de β-amyloïde, comprenant les éta pes:
- fractionnement d'un échantillon, comprenant Aβ et Aβ-biotinylé N-terminal ou seulement Aβ-biotinylé N-terminal est préparé et incubé, ainsi que des conformères différents de l'Aβ sont présents dans l'échantillon par agrégation,
- Aβ-conformère biotinylé de la fraction souhaité, que ne comprend pas des monomères d'Aβ, est immobilisé sur la surface d'un substrat présentant une forte affinité à la biotine,
- addition d'une sonde de contrôle de la qualité de l'agrégat,
- le comportement de liaison d'une sonde de contrôle de la qualité de l'agrégat au Aβ-conformère souhaité est dérivé par détermination des pa ramêtres cinétiques et/ou thermodynamiques à partir du signal de mesure,
- par lequel la qualité des agrégats immobilisés peut être verifiée,
- une substance à tester est ajoutée au conformère immobilisé et le compor tement de liaison de la substance à tester au Aβ-conformère souhaité est dérivé par détermination des paramêtres cinétiques et/ou thermodynamiques à partir du signal de mesure.

2. Procédé selon la revendication 1, **caractérisé en ce qu'**un échantillon avec des Aβ-conformères a été obtenu à partir d'une culture de cellule ou par prélèvement d'un corps.

3. Procédé selon l'une des revendications précédentes, **caractérisé par** une centrifugation à gradient de densité comme étape de fractionnement.

4. Procédé selon la revendication précédente, **caractérisé par** une calibration de la centrifugation à gradient de densité.

5. Procédé selon l'une des revendications précédentes, **caractérisé en ce qu'**un rapport des molécules biotinylés N-terminal aux molécules non biotinylés est chosi de 1 à 1:100.

6. Procédé selon l'une des revendications précédentes, **caractérisé par** le choix du fragment d'anticorps scFv-1C16 comme sonde de contrôle de la qualité de l'agrégat.

7. Procédé selon l'une des revendications précédentes, **caractérisé par** le choix des paramètres de test, où la sonde de contrôle de la qualité de l'agrégat dissocie de l'Aβ.

8. Procédé selon la revendication précédente, **caractérisé en ce que** la substance à tester dissocie de l'Aβ-conformère.

9. Procédé selon la revendication précédente, **caractérisé en ce que** la sonde de contrôle de la qualité de l'agrégat est de nouveau ajouté au conformère et la surface du substrat est vérifiée quant à la qualité pour d'autres études de liaison.

10. Procédé selon l'une des revendications précédentes, **caractérisé par** la réali sation d'une résonance plasmonique de surface ou d'une interférometrie biocouche pour la détermination des paramètres cinétiques et/ou ther mody namiques.

11. Procédé selon l'une des revendications précédentes, par lequel la première étape peut être omise, à condition que les Aβ-conformères souhaités puissent être obtenus par d'autres moyens, par example d'une autre expérience.
